# EUROPÄISCHE PATENTSCHRIFT

(11) **EP 0 664 285 B1**
(45) Veröffentlichungstag und Bekanntmachung des Hinweises auf die Patenterteilung: **23.04.1997**
(21) Anmeldenummer: 95100308.6
(22) Anmeldetag: 11.01.1995
(51) Int. Cl.: C07C 253/30, C07C 255/46

(54) **Verfahren zur Herstellung von 1-Amino-1-methyl-3(4)-cyano-cyclohexan**
Process for the preparation of 1-amino-1-methyl-3(4)-cyanocyclohexane
Procédé pour la préparation du amino-1-méthyl-3(4)-cyanocyclohexane

(30) Priorität: 24.01.1994 DE 4401929
(43) Veröffentlichungstag der Anmeldung: 26.07.1995
(73) Patentinhaber: BAYER AG, 51368 Leverkusen (DE)
(72) Erfinder: Arlt, Dieter Prof. Dr., D-51065 Köln (DE)

(56) Entgegenhaltungen:
- EP-A- 0 153 561
- DE-A- 1 965 004

## Beschreibung

Die Erfindung betrifft ein neues Verfahren zur Herstellung von 1-Amino-1-methyl-3(4)-cyano-cyclohexan (AMCC), das durch eine an sich bekannte Hydrierung in 1-Amino-1-methyl-3(4)-aminomethyl-cyclohexan (AMCA), dem Vorprodukt zur Herstellung von 1-Isocyanato-1-methyl-3(4)-isocyanatomethyl-cyclohexan (IMCI) überführt werden kann.

Aus der EP-A 0 153 561 ist bekannt, daß (IMCI) ein in der Polyurethanchemie vielseitig verwendbares, hochwertiges Diisocyanat darstellt und durch folgende schematisch dargestellte Synthesesequenz erhalten werden kann:

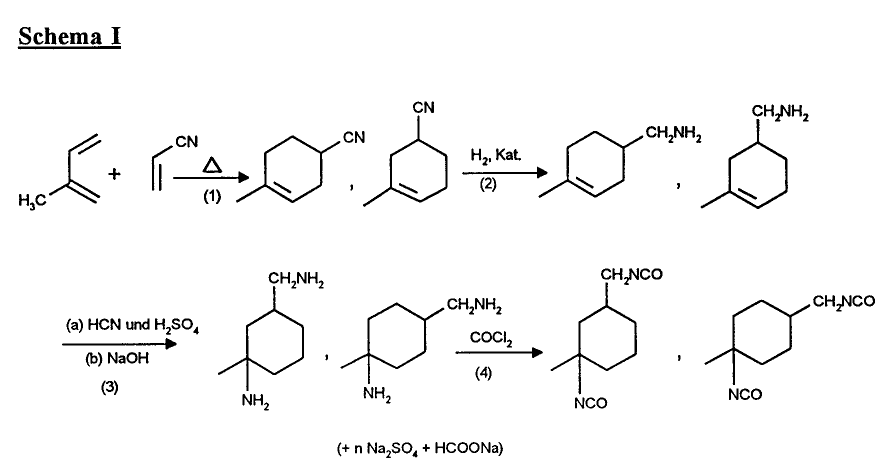

Ein Schlüsselschritt ist dabei die Ritterreaktion (3), die es ermöglicht, eine an ein tertiäres C-Atom gebundene Aminogruppe einzuführen. Diese Reaktion wird vorzugsweise in Schwefelsäure ausgeführt, wobei sich aus dem olefinischen Vorprodukt und Blausäure primär das entsprechende Formamid bildet, das anschließend hydrolysiert wird. Im Falle der hier konkret beschriebenen Umsetzung wird auf diesem Wege das Salz des AMCA gebildet, das im schwefelsauren Reaktionsgemisch gelöst vorliegt. Es wird - wie bislang bei der Aufarbeitung von Ritterreaktionsgemischen üblich - anschließend mit Alkali, z.B. Natronlauge, das Diamin freigesetzt und durch Extraktion mit geeigneten Lösungsmitteln von der wäßrigen Lösung der Alkalisalze abgetrennt.

Dabei fällt eine große Menge nicht verwertbarer Abfallsalze an, die für eine großtechnische Produktion unter Berücksichtigung der Umweltbelastung prohibitiv ist. Eine stöchiometrische Betrachtung der Herstellungsbeispiele (1a) und (1b) der EP-A-0 153 561 zeigt, daß im besten Falle mehr als 4 t eines Abfallgemisches von Na-sulfat und -formiat, im ungünstigeren Falle nahezu 9 t dieses Abfalles pro t AMCA anfallen.

Es war daher die der Erfindung zugrundeliegende Aufgabe einen neuen, von Abfallsalzen weitgehend befreiten Weg von den petrochemischen Ausgangsmaterialien Isopren und Acrylnitril zu IMCI zu eröffnen, d.h. ein Verfahren bereitzustellen, welches ohne die Bildung von Abfallsalzen die Herstellung von AMCC aus 4(5)-Cyano-1-methyl-cyclohexen (CMC) ermöglicht.

Diese Aufgabe konnte mit dem nachstehend näher beschriebenen erfindungsgemäßen Verfahren gelöst werden. Erfindungsgemäß erfolgt in der ersten Stufe a) die Umsetzung von 4(5)-Cyano-1-methyl-cyclohexen (CMC) mit Blausäure in Gegenwart von Schwefelsäure zum 1-Formamido-1-methyl-3(4)-cyano-cyclohexan (FMCC) und in der zweiten Stufe b) dessen selektive Hydrolyse zum AMCC:

Hier und im folgenden bedeutet "4(5)" bzw. "3(4)" das Vorliegen der Gemische aus den jeweiligen 4- und 5- bzw. 3- und 4-Isomeren.

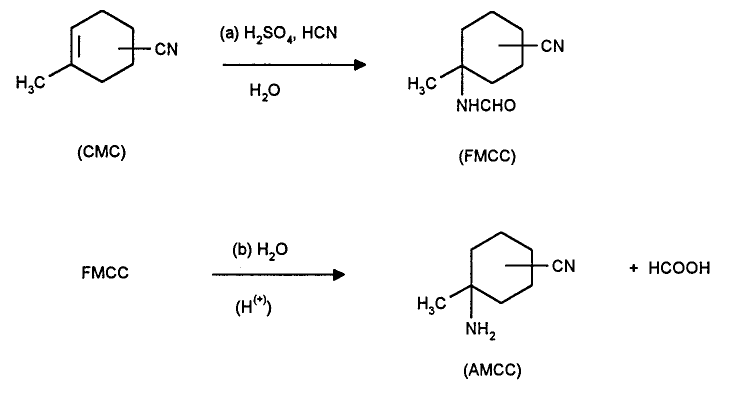

Gegenstand der Erfindung ist demzufolge ein Verfahren zur Herstellung von 1-Amino-1-methyl-3(4)cyano-cyclohexan (AMCC), dadurch gekennzeichnet, daß man
a) in einer ersten Reaktionsstufe 4(5)-Cyano-1-methyl-cyclohexen (CMC) mit überschüssiger Blausäure in Gegenwart von wäßriger, jedoch mindestens 50 Gew.-%iger Schwefelsäure in einer Menge von mindestens 1 Mol H₂SO₄ pro Mol CMC zu 1-Formamido-1-methyl-3(4)-cyano-cyclohexan (FMCC) umsetzt und das Reaktionsgemisch anschließend, gegebenenfalls unter vorhergehender und/oder gleichzeitiger Zugabe von Wasser, destillativ von überschüssiger Blausäure befreit und
b) in einer zweiten Stufe, das gemäß a) gebildete FMCC in Gegenwart von sauren Ionenaustauschern selektiv zu AMCC hydrolysiert in 0,5 bis 65 Gew.-%igen wäßrigen Ameisen- oder Schwefelsäurelösungen bei einer Gesamtmenge dieser Säuren von 1 bis 200 Mol-%, bezogen auf die Menge des vorliegenden FMCC bei 40 bis 150°C, gegebenenfalls unter Anwendung von Druck, durchführt und anschließend das gebildete AMCC nach erfolgter Neutralisation der vorliegenden Säure mit Ammoniak mit Lösungsmitteln aus dem Reaktionsgemisch extrahiert.

Das als Ausgangsmaterial eingesetzte CMC ist bekannt und in einfacher Weise durch eine Cycloadditionsreaktion aus Isopren und Acrylnitril herstellbar.

Die erste Stufe a) des erfindungsgemäßen Verfahrens besteht in der Umsetzung dieses Produkts mit Blausäure in wasserhaltiger Schwefelsäure. Diese Umsetzung wird in 50 bis 96 Gew.-%iger wäßriger Schwefelsäure durchgeführt, wobei in dem Reaktionsgemisch, bezogen auf die Menge des CMC, mindestens die stöchiometrisch erforderliche Menge Wasser vorliegen muß. Das Molverhältnis von Blausäure zu CMC liegt im allgemeinen bei 2:1 bis 20:1, vorzugsweise bei 8:1 bis 20:1. Pro Mol CMC liegt im Reaktionsgemisch mindestens 1 Mol H₂SO₄ vor. Vorzugsweise liegt das Molverhältnis von H₂SO₄ zu CMC bei 1,5:1 bis 3:1. Die Umsetzung erfolgt im allgemeinen bei 0 bis 100°C, entsprechend einem Druck von ca. 1 bis 10 bar. Die Reaktionszeit für die Umsetzung ist sowohl von der gewählten Reaktionstemperatur als auch von der Schwefelsäure- und Blausäure-Konzentration abhängig und liegt im Bereich einiger Minuten bis Stunden. Im allgemeinen wird das CMC zu einer Mischung aus Schwefel- und Blausäure zudosiert, wobei z.B. durch Rühren der Reaktionsmischung für eine gute Vermischung gesorgt werden sollte. Die Umsetzung kann sowohl diskontinuierlich, z.B. in einem Rührkessel, als auch kontinuierlich, z.B. in einer Rührkesselkaskade oder einem Rohrreaktor, ausgeführt werden. Die exotherme Reaktionswärme kann durch entsprechende Kühleinrichtungen abgeführt werden, z.B. durch Rückflußkühlung der siedenden Blausäure. In einer besonderen Ausführongsform wird diese Reaktionsstufe adiabatisch durchgeführt und die Reaktionswärme zur anschließenden Verdampfung überschüssiger Blausäure genutzt. Diese Reaktion ist im Prinzip bereits bekannt (DE-OS 1 965 004).

Nach Beendigung der ersten Stufe a) des erfindungsgemäßen Verfahrens wird zunächst die im Überschuß vorliegende Blausäure, gegebenenfalls unter vorhergehender und/oder gleichzeitiger Zugabe von Wasser im Hinblick auf die Weiterbehandlung der wäßrig-sauren Reaktionsmischung in verdünnter Form, destillativ abgetrennt und an den Prozeßanfang zurückgeführt. Das in dem so erhaltenen Reaktionsgemisch vorliegende FMCC wird dann entsprechend der zweiten Stufe des erlindungsgemäßen Verfahrens weiterbehandelt. Diese zweite Stufe b) des erfindungsgemäßen Verfahrens kann nach verschiedenen Varianten erfolgen. Allen diesen Varianten gemeinsam ist die selektive Hydrolyse des FMCC zu AMCC in wäßriger, saurer Phase. Der Umstand, daß diese unter Abspaltung der Formylgruppe ablaufende selektive Hydrolyse in guten Ausbeuten durchführbar ist, muß als überraschend bezeichnet werden, da es zum Standardwissen des Fachmanns zählt (siehe z.B. C. Ferri, Reaktionen d. org. Synthese, Georg Thieme Verlag Stuttgart 1978, S. 202 und dort angegebene weitere Literatur), daß Nitrile in saurem Milieu zu Carbonsäuren bzw. Carbonsäureamiden hydrolisiert werden und daß somit zu erwarten war, daß bei einer Hydrolyse des Formamidonitrils als Reaktionsprodukt, die entsprechende Aminocarbonsäure bzw. dessen Amid erhalten würde.

Allen nachstehend näher beschriebenen Varianten der zweiten Stufe des erfindungsgemäßen Verfahrens gemeinsam ist ferner, daß sie in einem wäßrigsauren Reaktionsmedium bei 40 bis 150°C, gegebenenfalls unter entsprechender Anwendung von Druck, durchgeführt werden, wobei als Säuren vorzugsweise Schwefelsäure, aber auch Ameisensäure oder sauer eingestellte Ionenaustauscher bzw. gleichzeitig mehrerer derartiger Säuren in Betracht kommen. Die Säurekonzentration in dem wäßrig-sauren Reaktionsmedium kann hierbei innerhalb weiter Bereiche von beispielsweise 0,5- bis 65-, vorzugsweise 0,5- bis 50 gew.-%igen wäßrigen Säuren schwanken, wobei im allgemeinen, bezogen auf die Menge des eingesetzen FMCC die Menge der Säure bei ca. 1 bis 200 Mol-% liegt.

Gemäß der ersten Variante des erfindungsgemäßen Verfahrens erfolgt die zweite Stufe b) direkt im Anschluß an die destillative Entfernung der überschüssigen Blausäure ohne Zwischenisolierung des FMCC, gegebenenfalls nach (weiterem) Verdünnen der schwefelsauren Reaktionslösung der ersten Stufe auf eine Säurekonzentration, bezogen auf im Reaktionsgemisch vorliegende Schwefelsäure und Wasser, ohne Einbeziehung anderer Bestandteile, von bis zu 65 Gew.-%, vorzugsweise 0,5 bis 50 Gew.-% und Durchführung der Hydrolyse innerhalb des oben genannten Temperaturbereichs und beim genannten Säurekonzentrationsbereich. Im Anschluß an die selektive Hydrolyse unter Abspaltung der Formylgruppe wird die im Reaktionsgemisch vorliegende Säure mit Ammoniak, vorzugsweise wäßrigem Ammoniak neutralisiert und anschließend das AMCC mit geeigneten Lösungsmitteln extrahiert.

Für diese Extraktion geeignete Lösungsmittel sind beispielsweise chlorierte Kohlenwasserstoffe wie Dichlormethan oder Chlorbenzol, Ether wie z.B. tert.-Butylmethylether, Ester wie z.B. Ethyl- oder n-Butylacetat, Ketone wie z.B. Methylethylketon oder Cyclohexanon, Alkohole wie z.B. n- und i-Butanol, 1-Pentanol, 2-Methyl-1-butanol und 2-Ethyl-hexanol oder Gemische derartiger Lösungsmittel.

Nach der Extraktion kann das Lösungsmittel durch Vakuumdestillation von der organischen Phase abgetrennt und wiederverwendet werden. Das rohe AMCC kann, gewünschtenfalls, von geringfügigen Verunreinigungen durch Destillation befreit werden. Es ist jedoch auch möglich, das als Verfahrensprodukt anfallende, rohe AMCC einer weiteren Verwertung (Hydrierung zu AMCA) zuzuführen, bzw. die bei der genannten Extraktion anfallende organische Phase ohne Abdestillieren des Lösungsmittels direkt hierbei zu verwenden, vorausgesetzt zur Extraktion wurden Lösungsmittel verwendet, die sich sowohl für die Extraktion als auch für die Hydrierung eignen, und die dann erst nach der Hydrierung und Rückgewinnung in die Extraktionsstufe zurückgeführt werden. Auf diese Weise ist selbstverständlich eine Minimierung des Aufarbeitungsaufwands und Lösungsmittelverbrauchs möglich.

Die bei der genannten Extraktion anfallende wäßrig-saure Phase kann der nachstehend noch beschriebenen Aufarbeitung zugeführt werden.

Bei der zweiten Variante des erfindungsgemäßen Verfahrens wird zur Durchführung der zweiten Stufe b) das in der ersten Stufe anfallende, von überschüssiger Blausäure befreite Reaktionsgemisch gegebenenfalls mit (weiterem) Wasser versetzt, so daß der Schwefelsäureanteil des Gemisches bei 20 bis 70 Gew.-% liegt. Aus diesem wäßrig-sauren Reaktionsgemisch wird anschließend das FMCC mit Lösungsmitteln der bereits oben beispielhaft genannten Art extrahiert. Das dann aus der Lösungsmittelphase durch destillative Entfernung des Lösungsmittels gewonnene FMCC kann als Rohprodukt oder auch nach destillativer Reindarstellung (Vakuumdestillation) in der nachfolgenden Hydrolysestufe eingesetzt werden. Die hierbei zur Anwendung gelangenden Reaktionsbedingungen (Art und Konzentration der eingesetzten Säuren, Reaktionstemperatur) entsprechen den oben gemachten Ausführungen.

Da bei der Hydrolyse des FMCC zu AMCC eine äquivalente Menge Ameisensäure entsteht, bleibt die wäßrige Phase stets sauer. Aus diesem Grund ist es möglich, zur Hydrolyse lediglich eine katalytisch wirksame Menge an Schwefelsäure einzusetzen, so daß nach nachfolgender Neutralisation mit Ammoniak und Extraktion des gebildeten AMCC mit Lösungsmitteln der bereits beispielhaft genannten Art in der resultierenden wäßrigen Phase als Salz vor allem Ammoniumformiat vorliegt, welches gegebenenfalls getrennt isoliert werden kann. Bei dieser Variante des erfindungsgemäßen Verfahrens kann selbstverständlich ein geringer Teil der nach der ersten Extraktion anfallenden wäßrigen Phase als Katalysator für die Hydrolyse verwendet werden und eine diesem Teil entsprechende (frische) Schwefelsäuremenge bei einem nächsten Reaktionszyklus am Prozeßbeginn eingesetzt werden. Auf diese Weise kann eine unerwünschte Anreicherung von Nebenprodukten im Reaktionsmedium vermieden werden. Da bei der zweiten Variante des erfindungsgemäßen Verfahrens eine Neutralisation der in der ersten Stufe eingesetzten Schwefelsäure nicht erfolgt und in der zweiten Stufe, wie ausgeführt, lediglich katalytische Schwefelsäuremengen zur Anwendung gelangen müssen, gestattet diese Variante des erfindungsgemäßen Verfahrens eine Minimierung der Menge der zur Aufarbeitung anfallenden Salze. Wegen der vergleichsweise geringen Menge an zu neutralisierender Säure könnte diese Neutralisation selbstverständlich auch unter Verwendung von alkalischen Neutralisationsmitteln wie beispielsweise Natronlauge durchgeführt werden, falls die hierbei anfallenden vergleichsweise geringen Mengen an Alkalisalzen in Kauf genommen werden können.

Zur Isolierung des als Verfahrensprodukt vorliegenden AMCC wird das wäßrigsaure nach der Hydrolyse vorliegende Reaktionsgemisch mit Ammoniak neutralisiert und das AMCC mit Lösungsmitteln der beispielhaft genannten Art extrahiert. Die weitere Aufarbeitung bzw. Verwertung der hierbei anfallenden, das AMCC enthaltenden organischen Phase entspricht den oben zur ersten Variante gemachten Ausführungen.

Die nach der Extraktion vorliegende wäßrige Phase kann gegebenenfalls zur Herstellung von Ammoniumformiat aufgearbeitet werden, oder auch beispielsweise entsprechend den weiter unten gemachten Ausführungen aufgearbeitet werden.

Gemäß der dritten Variante des erfindungsgemäßen Verfahrens wird das wäßrigsaure, von überschüssiger Blausäure befreite und gegebenenfalls mit Wasser verdünnte Reaktionsgemisch der ersten Stufe mit Ammoniak neutralisiert und gegebenenfalls mit einer weiteren Menge Wasser versetzt, so daß das Gemisch einen Gehalt an Ammoniumsalzen von ca. 20 bis 70 Gew.-% aufweist. Anschließend wird das in dem Gemisch vorliegende FMCC mit Lösungsmitteln der beispielhaft genannten Art extrahiert und entsprechend den Ausführungen zur zweiten Variante weiterverarbeitet. Obwohl bei dieser Variante des erfindungsgemäßen Verfahrens wegen der Neutralisation der Gesamtmenge der in der ersten Stufe eingesetzten Schwefelsäure eine erhebliche Menge an zur Aufarbeitung anstehenden Ammoniumsalze gebildet wird, handelt es sich bei dieser Variante um eine bevorzugte Arbeitsweise, da die Extraktion auch aus relativ konzentrierten Lösungen besonders vollständig erfolgt und dadurch die für die Aufarbeitung aufzuwendende Energie verringert wird.

Alle Extraktionen können in an sich bekannter Weise, z.B. in Misch- und Trennbehältern, vorzugsweise kontinuierlich, z.B. in Kaskaden von hintereinander geschalteten Misch- und Trennbehältern oder in Extraktionskolonnen durchgeführt werden. Hierbei ist insbesondere überraschend, daß es möglich ist, das im Vergleich zu Ammoniak eine höhere Basisität aufweisende AMCC aus den durch Neutralisation mit Ammoniak gebildeten ammoniakalischen Lösungen in guten Ausbeuten zu extrahieren. Diese Extrahierbarkeit war jedoch neben der nicht zu erwartenden Möglichkeit einer selektiven Hydrolyse des FMCC eine Voraussetzung für die Durchführbarkeit des erfindungsgemäßen Verfahrens.

Die bei den einzelnen Extraktionen anfallenden wäßrigen Phasen können, falls es sich um Salzlösungen handelt, selbstverständlich unter Gewinnung der in ihnen gelösten Salze aufgearbeitet werden. Vorzugsweise erfolgt die Aufarbeitung der einzelnen wäßrigen Phase jedoch getrennt oder gemeinsam unter Gewinnung von am Prozeßanfang wiederverwendbarer konzentierter Schwefelsäure bzw. von zur Herstellung von Schwefelsäure geeignetem Schwefeldioxid. Dies bedeutet, daß die Aufarbeitung von verdünnten Schwefelsäurelösungen lediglich in einem Aufkonzentrieren auf die gewünschte Konzentration besteht, während die Aufarbeitung von wäßrigen, gegebenenfalls überschüssige Schwefelsäure enthaltenden Ammoniumsulfat-und/oder Ammoniumformiat-Lösungen in einer an sich bekannten über die Stufe des Aufkonzentrierens hinausgehenden thermischen Spaltungen unter Freisetzung von Stickstoff, Wasser, gegebenenfalls Kohlendioxid, sowie Schwefeldioxid besteht. Diese thermische Spaltung erfolgt im allgemeinen bei Temperaturen von >1000°C.

Das nach den verschiedenen Varianten des erfindungsgemäßen Verfahrens erhaltene AMCC kann in an sich bekannter Weise zu AMCA hydriert werden, welches seinerseits durch eine an sich bekannte Phosgenierung in IMCI überführt werden kann.

In den nachfolgenden Beispielen beziehen sich alle Prozentangaben auf das Gewicht.

### Beispiel 1 (Stufe a))

Zu einer Mischung von 108 g Wasser, 600 g 96 %iger Schwefelsäure und 900 ml Blausäure werden bei 27 bis 29°C innerhalb von 1,5 Stunden gleichmäßig mit einer Dosierpumpe unter Rühren 363 g (=3 Mol) 4(5)-Cyano-1-methyl-cyclohexen (Isomerengemisch, erhalten durch Cycloaddition von Isopren und Acrylnitril) zudosiert, wobei die Reaktionswärme durch Rückflußkühlung der siedenden Blausäure abgeführt wird. 10 Minuten nach Ende der Eduktzugabe werden 546 g Wasser zugegeben und gleichzeitig die überschüssige Blausäure abdestilliert, zunächst unter Normaldruck, gegen Ende bei vermindertem Druck. Dann werden bei 20 bis 30°C unter Rühren und Kühlen mit Eiswasser 816 g 25 %ige wäßrige NH₃-Lösung zudosiert. Die ammoniakalische Reaktionslösung wird anschließend dreimal mit je 1 000 ml Dichlormethan extrahiert. Man erhält nach Entfernung des Lösungsmittels im Rotationsverdampfer 521,5 g rohes 1-Formamido-1-methyl-3(4)-cyanocyclohexan-Isomerengemisch nach GC 90 %ig, d.h. 469 g Reinprodukt (= 94,2 % d.Th.)

### Beispiel 2 (Stufen a) und b), 1. Variante)

Zu einer Mischung von 108 g Wasser, 600 g 96 %iger Schwefelsäure und 1 200 ml Blausäure werden bei 27 bis 29°C innerhalb von 90 Minuten gleichmäßig mit einer Dosierpumpe unter Rühren des Reaktionsgemisches 363 g (=3 Mol) 4(5)-Cyano-1-methyl-cyclohexen zudosiert, wobei die Reaktionswärme durch Rückflußkühlung der siedenden Blausäure abgeführt wird 10 Minuten nach Ende der Eduktzugabe werden 792 g Wasser zugegeben und gleichzeitig wird die überschüssige Blausäure abdestilliert. Danach wird die Reaktionsmischung 300 Minuten auf 60°C erwärmt und anschließend unter Kühlung auf 20 bis 30°C durch Zugabe von 1 100 g 25 %iger wäßriger NH₃-Lösung ammoniakalisch gestellt und anschließend dreimal mit je 1 000 ml Dichlormethan extrahiert. Man erhält nach Entfernung des Lösungsmittels im Rotationsverdampfer 380 g Rohprodukt und nach Destillation im Dünnschichtverdampfer 350 g (=84 % d.Th.) reines 1-Amino-1-methyl-3-(4)-cyano-cyclohexan (Isomerengemisch).

### Beispiel 3 (Stufe b))

5,0 g 1-Formamido-1-methyl-3(4)-cyano-cyclohexan werden unter Rühren in 12 g 48 %iger Schwefelsäure gelöst. Die Mischung wird 165 Minuten auf 80°C erwärmt, dann werden unter Kühlung auf 20°C 27 ml wäßrige Ammoniaklösung (25 % NH₃-Gehalt) hinzugegeben. Die wäßrige Phase wird anschließend dreimal mit je 25 ml Dichlormethan extrahiert. Man erhält nach den Einengen am Rotationsverdampfer 3,0 g (=72,2 % d.Th.) eines reinen Isomerengemisches von 1-Amino-1-methyl-3-(4)-cyanocyclohexanen.

### Beispiel 4 (Stufe b))

44,9 g 1-Formamido-1-methyl-3(4)-cyano-cyclohexan werden unter Rühren in 120 g 48 %iger Schwefelsäure zudosiert. Die Mischung wird anschließend 90 Minuten auf 80°C erwärmt, nachfolgend durch Zugabe von 110 g 25 %iger NH₃-Lösung ammoniakalisch gestellt und anschließend zweimal mit je 250 ml Dichlormethan extrahiert. Man erhält nach den Einengen am Rotationsverdampfer 30,7 g von isomeren 1-Amino-1-methyl-3-(4)-cyanocyclohexanen, die nach GC-Analyse noch 1,9 % nicht umgesetzte Edukte enthalten. Das entspricht einer Ausbeute von 80,6 % d.Th. an erwünschten Aminonitrilen.

### Beispiel 5 (Stufe b))

432 g 1-Formamido-1-methyl-3(4)-cyano-cyclohexan werden unter Rühren in 1 500 g 38,4 %iger Schwefelsäure zudosiert.. Dann wird die Mischung 5 Stunden und 35 Minuten auf 60°C erwärmt. Anschließend wird durch Zugabe von 1 100 g 25 %iger NH₃-Lösung die Lösung bei 20°C ammoniakalisch gestellt. Das Reaktionsprodukt wird dreimal mit je 1 000 ml Dichlormethan aus der Wasserphase extrahiert. Nach dem Einengen am Rotationsverdampfer erhält man 357 g (99,4 % d.Th.) eines Gemisches von isomeren 1-Amino-1-methyl-3-(4)-cyano-cyclohexanen.

### Beispiel 6 (Stufe b))

Eine Mischung von 25 g 1-Formamido-1-methyl-3(4)-cyano-cyclohexan und 100 ml 1 %iger wäßriger Schwefelsäure wird 74 Stunden unter Rühren auf 100°C erwärmt. Dann wird die Reaktionsmischung im Vakuumrotationsverdampfer eingeengt. Das erhaltene Konzentrat (37,6 g) wird durch Zugabe von 12 ml 25 %iger wäßriger NH₃-Lösung ammoniakalisch gestellt und anschließend dreimal mit je 50 ml Dichlormethan extrahiert. Man erhält nach dem Einengen der organischen Phase im Rotationsverdampfer 19,9 g einer Mischung, die nach GC-Analyse 71,3 % 1-Amino-1-methyl-3(4)-cyano-cyclohexan und 27,8 % 1-Formamido-1-methyl-3(4)-cyano-cyclohexan enthält. Das Produktgemisch kann durch fraktionierte Destillation im Vakuum aufgetrennt und der erhaltene Edukt-Anteil zur Hydrolyse zurückgeführt werden.

### Beispiel 7 (Stufe b))

Eine Mischung von 50 g 1-Formamido-1-methyl-3(4)-cyano-cyclohexan, 200 g Wasser und 5 g saurem Ionenaustauscher (Dowex 50 WX 8) werden unter Rühren 90 Stunden auf 100°C erwärmt. Der Ionenaustauscher wird danach abfiltriert das Filtrat im Vakuumrotationsverdampfer aufkonzentriert. Das Konzentrat wird mit 50 ml 25 %iger wäßriger NH₃-Lösung versetzt und dreimal mit je 150 ml Dichlormethan extrahiert. Man erhält nach dem Einengen der organischen Phase 42 g einer Mischung, die aus 64,8 % AMCC und 25,2 % FMCC besteht, d.h. die Reaktion ist mit einer Selektivität von 100 % verlaufen. Die wäßrige Phase enthält ausschließlich Ammoniumformiat.

### Beispiel 8 (Stufen a) und b), 2. Variante)

a) Zu einer Mischung von 108 g Wasser, 600 g 96 %iger Schwefelsäure und 900 ml Blausäure werden bei 27 bis 29°C innerhalb von 1,5 Stunden gleichmäßig mit einer Dosierpumpe unter Rühren 363 g (= 3 Mol) 4(5)-Cyano-1-methyl-cyclohexen (Isomerengemisch, erhalten durch Cycloaddition von Isopren und Acrylnitril) zudosiert, wobei die Reaktionswärme durch Rückflußkühlung der siedenden Blausäure abgeführt wird. 10 Minuten nach Ende der Eduktzugabe werden 546 g Wasser zugegeben und anschließend die überschüssige Blausäure im Vakuum abdestilliert, wobei die Temperatur der Reaktionsmischung auf 30°C gehalten wird. Dann wird die Reaktionsmischung durch Zugabe von 800 g Wasser weiter verdünnt und anschließend dreimal mit je 1 l Dichlormethan extrahiert.
   Man erhält nach Entfernung des Dichlormethans im Rotationsverdampfer 471 g rohes 1-Formamido-1-methyl-3(4)-cyano-cyclohexan-Isomerengemisch nach GC-Analyse 93,8 %ig.
   Die wäßrige Phase wird in einem Dünnschichtverdampfer bei 50°C Wandtemperatur im Vakuum aufkonzentriert. Man erhält 762 g Konzentrat, dem 76 g entnommen werden, die einer thermischen Spaltung zur Wiedergewinnung von SO₂ zugeführt werden können.
   Anschließend wird die Restmenge des Konzentrates durch 76 g frische 80 %ige Schwefelsäure ergänzt und erneut als saure Reaktionsphase in die Stufe a) des Verfahrens eingesetzt, wobei nach Zugabe von 900 ml Blausäure unter den oben angegebenen Bedingungen 302,5 g CMC umgesetzt werden. Nach der Aufarbeitung unter den angegebenen Bedingungen erhält man 388 g rohes 1-Formamido-1-methyl-3(4)-cyano-cyclohexan-Isomerengemisch.
b) 471 g des nach Stufe a) erhaltenen 1-Formamido-1-methyl-3(4)-cyano-cyclohexan-Isomerengemisches werden mit 76 g des in Stufe a) erhaltenen Schwefelsäurekonzentrates und 250 g Wasser vermischt und 30 Stunden auf 100°C erwärmt. Danach wird die Lösung durch Zugabe von 85 g 25 %iger wäßriger NH₃-Lösung neutralisiert und anschließend durch Abdestillieren von Wasser im Vakuumrotationsverdampfer aufkonzentriert. Der dabei erhaltene Feststoff wird in 353 g 21 %iger wäßriger NH₃-Lösung gelöst und anschließend dreimal mit je 1000 ml Dichlormethan extrahiert. Man erhält nach dem Abdestillieren des Extraktionsmittels 361 g rohes AMCC, nach GC 94 %ig (= 87 % d.Th.).

## Patentansprüche

1. Verfahren zur Herstellung von 1-Amino-1-methyl-3(4)-cyano-cyclohexan (AMCC), dadurch gekennzeichnet, daß man
a) in einer ersten Reaktionsstufe 4(5)-Cyano-1-methyl-cyclohexen (CMC) mit überschüssiger Blausäure in Gegenwart von wäßriger, jedoch mindestens 50 Gew.-%iger Schwefelsäure in einer Menge von mindestens 1 Mol H₂SO₄ pro Mol CMC zu 1-Formamido-1-methyl-3(4)-cyano-cyclohexan (FMCC) umsetzt und das Reaktionsgemisch anschließend, gegebenenfalls unter vorhergehender und/oder gleichzeitiger Zugabe von Wasser, destillativ von überschüssiger Blausäure befreit und
b) in einer zweiten Stufe, das gemäß a) gebildete FMCC in Gegenwart von sauren Ionenaustauschern selektiv hydrolysiert in 0,5 bis 65 Gew.%igen wäßrigen Ameisen- oder Schwefelsäurelösungen bei einer Gesamtmenge dieser Säuren von 1 bis 200 Mol-%, bezogen auf die Menge des vorliegenden FMCC bei 40 bis 150°C, gegebenenfalls unter Anwendung von Druck, durchführt und anschließend das gebildete AMCC nach erfolgter Neutralisation der vorliegenden Säure mit Ammoniak mit Lösungsmitteln aus dem Reaktionsgemisch extrahiert.

2. Verfahren gemäß Anspruch 1, dadurch gekennzeichnet, daß man das gemäß Stufe a) erhaltene, von überschüssiger Blausäure befreite Reaktionsgemisch gegebenenfalls durch Zugabe von Wasser vor, während und/oder nach der Entfernung der Blausäure bis auf einen Schwefelsäuregehalt des Gemisches, bezogen auf Schwefelsäure und Wasser ohne Einbeziehung anderer Bestandteile von bis zu 65 Gew.-% verdünnt, durch anschließendes Erhitzen des so erhaltenen Gemischs auf 40 bis 150°C die Hydrolyse von FMCC zu AMCC bewirkt, dann die vorliegende Schwefelsäure mit Ammoniak neutralisiert und das gebildete AMCC durch Extraktion mit Lösungsmitteln aus der Wasserphase gewinnt.

3. Verfahren gemäß Anspruch 1, dadurch gekennzeichnet, daß man aus dem gemaß Stufe a) erhaltenen, von überschüssiger Blausäure befreiten Reaktionsgemisch, gegebenenfalls nach Zugabe von weiterem Wasser, das gebildete FMCC durch Extraktion mit Lösungsmitteln isoliert und der Stufe b) zuführt.

4. Verfahren gemäß Anspruch 1, dadurch gekennzeichnet, daß man die in dem von überschüssiger Blausäure befreiten Reaktionsgemisch gemäß Stufe a) vorliegende Schwefelsäure durch Zugabe von Ammoniak, gegebenenfalls unter vorhergehender, gleichzeitiger und/oder anschließender Zugabe von weiterem Wasser neutralisiert, daß in dem Gemisch vorliegende FMCC durch Extraktion mit Lösungsmitteln abtrennt und der Stufe b) zuführt.

5. Verfahren gemaß Anspruch 1 bis 4, dadurch gekennzeichnet, daß man die von FMCC oder AMCC durch Extraktion befreiten, Schwefelsäure und/oder Ammoniumsulfat und/oder Ammoniumformiat enthaltenden Phasen, gegebenenfalls als Gemisch nach üblichen Verfahren zu in der Stufe a) wiederverwendbarer Schwefelsäure aufkonzentriert oder unter Abspaltung von zur Herstellung von Schwefelsäure wiederverwendbarem Schwefeldioxid einer thermolytischen Spaltung unterzieht.

6. Verfahren gemaß Anspruch 1 zur Herstellung von 1-Amino-1-methyl-3(4)-cyano-cyclohexan (AMCC), dadurch gekennzeichnet, daß man 1-Formamido-1-methyl-3-(4)-cyano-cyclohexan (FMCC) in saurer, wäßriger Phase bei 40 bis 150°C zu AMCC hydrolysiert und dieses anschließend nach Neutralisation der im Reaktionsgemisch vorliegenden Säure mit Ammoniak mit organischen Lösungsmitteln aus dem Reaktionsgemisch extrahiert.

7. Verfahren gemäß Anspruch 6, dadurch gekennzeichnet, daß man die Hydrolyse des FMCC in 0,5 bis 50 Gew.-%iger wäßriger Schwefelsäure durchführt.

## Claims

1. Process for the production of 1-amino-1-methyl-3(4)-cyanocyclohexane (AMCC), characterised in that
a) in a first reaction stage, 4(5)-cyano-1-methylcyclohexene (CMC) is reacted with excess hydrocyanic acid in the presence of aqueous, but at least 50 wt.% sulphuric acid in a quantity of at least 1 mol of H₂SO₄ per mol of CMC to yield 1-formamido-1-methyl-3(4)-cyanocyclohexane (FMCC) and excess hydrocyanic acid is removed from the reaction mixture by distillation, optionally with preceding and/or simultaneous addition of water and
b) in a second stage, the FMCC formed according to a) is selectively hydrolysed in the presence of acidic ion exchangers in 0.5 to 65 wt.% aqueous formic or sulphuric acid solutions at a total quantity of these acids of 1 to 200 mol.%, relative to the quantity of FMCC present, at 40 to 150°C, optionally with application of pressure, and then, once the acid present has been neutralised with ammonia, the AMCC formed is extracted from the reaction mixture with solvents.

2. Process according to claim 1, characterised in that the reaction mixture obtained according to stage a), from which excess hydrocyanic acid has been removed, is optionally diluted by the addition of water before, during and/or after removal of the hydrocyanic acid to a sulphuric acid content of the mixture, relative to sulphuric acid and water without including other constituents, of up to 65 wt.%, the resultant mixture is subsequently heated to 40 to 150°C to hydrolyse the FMCC to yield AMCC, the sulphuric acid present is then neutralised with ammonia and the AMCC formed is recovered from the aqueous phase by extraction with solvents.

3. Process according to claim 1, characterised in that the FMCC formed is isolated, optionally after the addition of further water, by extraction with solvents from the reaction mixture obtained according to stage a), from which excess hydrocyanic acid has been removed, and introduced into stage b).

4. Process according to claim 1, characterised in that the sulphuric acid present in the reaction mixture according to stage a), from which excess hydrocyanic acid has been removed, is neutralised by the addition of ammonia, optionally with preceding, simultaneous or subsequent addition of further water and that the FMCC present in the mixture is separated by extraction with solvents and introduced into stage b).

5. Process according to claims 1 to 4, characterised in that the phases containing sulphuric acid and/or ammonium sulphate and/or ammonium formate from which FMCC or AMCC have been removed by extraction, optionally as a mixture, are concentrated using conventional processes to yield sulphuric acid reusable in stage a) or are subjected to thermolytic decomposition with elimination of sulphur dioxide which may be reused for the production of sulphuric acid.

6. Process according to claim 1 for the production of 1-amino-1-methyl-3(4)-cyanocyclohexane (AMCC), characterised in that 1-formamido-1-methyl-3(4)-cyanocyclohexane (FMCC) is hydrolysed in an acidic, aqueous phase at 40 to 150°C to yield AMCC and, once the acid present in the reaction mixture has been neutralised with ammonia, this is extracted from the reaction mixture with organic solvents.

7. Process according to claim 6, characterised in that the FMCC is hydrolysed in 0.5 to 50 wt.% aqueous sulphuric acid.

## Revendications

1. Procédé de préparation du 1-amino-1-méthyl-3(4)-cyanocyclohexane (AMCC) caractérisé en ce que
a) dans un premier stade de réaction, on fait réagir le 4(5)-cyano-1-méthylcyclohexène (CMC) avec un excès d'acide cyanhydrique en présence d'un acide sulfurique aqueux mais à une concentration d'au moins 50 % en poids, en quantité d'au moins 1 mol d'H₂SO₄ par mole de CMC, la réaction donnant le 1-formamido-1-méthyl-3(4)-cyanocyclohexane (FMCC), et on débarrasse le mélange de réaction de l'excès d'acide cyanhydrique par distillation, éventuellement après addition d'eau et/ou avec addition simultanée d'eau, et
b) dans un deuxième stade, on soumet le FMCC formé en a) ci-dessus à hydrolyse sélective en présence d'échangeurs d'ions acides, dans des solutions aqueuses d'acide formique ou d'acide sulfurique à des concentrations de 0,5 à 65 % en poids, la quantité totale de ces acides représentant de 1 à 200 mol % par rapport à la quantité du FMCC présent, à des températures de 40 à 150°C, le cas échéant sous pression puis, après neutralisation de l'acide présent par l'ammoniac, on extrait l'AMCC formé du mélange de réaction par extraction à l'aide de solvants.

2. Procédé selon la revendication 1, caractérisé en ce que l'on dilue le cas échéant le mélange de réaction obtenu au stade a), débarrassé de l'excès d'acide cyanhydrique, par addition d'eau avant, durant ou après l'élimination de l'acide cyanhydrique, jusqu'à une teneur en acide sulfurique du mélange, rapportée à l'acide sulfurique et à l'eau non tenu compte des autres constituants, qui peut aller jusqu'à 65 % en poids, on provoque l'hydrolyse du FMCC en AMCC par chaufface subséquent du mélange à des températures de 40 à 150°C, on neutralise ensuite l'acide sulfurique présent par l'ammoniac et on isole l'AMCC formé de la phase aqueuse par extraction à l'aide de solvants.

3. Procédé selon la revendication 1, caractérisé en ce que, à partir du mélange de réaction obtenu au stade a), débarrassé de l'acide cyanhydrique en excès, le cas échéant après addition de compléments d'eau, on isole le FMCC formé par extraction à l'aide de solvants et on exécute le stade b).

4. Procédé selon la revendication 1, caractérisé en ce que l'on neutralise l'acide sulfurique présent dans le mélange de réaction du stade a), débarrassé de l'excès d'acide cyanhydrique, par addition d'ammoniac, le cas échéant après addition de compléments d'eau ou avec addition simultanée et/ou subséquente de compléments d'eau, en ce que l'on sépare le FMCC présent dans le mélange par extraction à l'aide de solvants et en ce qu'on l'envoie au stade b).

5. Procédé selon les revendications 1 à 4, caractérisé en ce que les phases débarrassées du FMCC ou de l'AMCC par extraction et contenant de l'acide sulfurique et/ou du sulfate d'ammonium et/ou du formiate d'ammonium, le cas échéant à l'état de mélange, sont concentrées par des techniques usuelles en acide sulfurique réutilisable au stade a) ou bien soumises à une scission à la chaleur avec libération de dioxyde de soufre réutilisable pour la préparation d'acide sulfurique.

6. Procédé selon la revendication 1 pour la préparation du 1-amino-1-méthyl-3(4)-cyanocyclohexane (AMCC), caractérisé en ce que l'on hydrolyse le 1-formamido-1-méthyl-3(4)-cyanocyclohexane (FMCC) en AMCC en phase aqueuse acide à des températures de 40 à 150°C et après neutralisation de l'acide présent dans le mélange de réaction par l'ammoniac, on extrait l'AMCC du mélange de réaction à l'aide de solvants organiques.

7. Procédé selon la revendication 6, caractérisé en ce que l'on hydrolyse le FMCC dans de l'acide sulfurique aqueux à une concentration de 0,5 à 50 % en poids.
